# EUROPÄISCHE PATENTSCHRIFT

(11) **EP 1 797 025 B1**
(45) Veröffentlichungstag und Bekanntmachung des Hinweises auf die Patenterteilung: **13.08.2008**
(21) Anmeldenummer: 05787173.3
(22) Anmeldetag: 26.09.2005
(51) Int. Cl.: C07C 35/08, C11B 9/00

(54) **4-ISOAMYLCYCLOHEXANOL ALS RIECHSTOFF**
4-ISOAMYLCYCLOHEXANOL AS ODIFERANT
4-ISOAMYLCYCLOHEXANOL COMME SUBSTANCE ODORANTE

(30) Priorität: 30.09.2004 DE 102004047536
(43) Veröffentlichungstag der Anmeldung: 20.06.2007
(73) Patentinhaber: Symrise GmbH & Co. KG, 37603 Holzminden (DE)
(72) Erfinder: RECKZIEGEL, Aurelia, 41540 Dormagen (DE); SURBURG, Horst, 37603 Holzminden (DE); DILK, Erich, 37603 Holzminden (DE)
(74) Vertreter: Stilkenböhmer, Uwe Michael
(86) Internationale Anmeldenummer: PCT/EP2005/054810
(87) Internationale Veröffentlichungsnummer: WO 2006/035010

(56) Entgegenhaltungen:
- US-A- 4 400 545
- DAVID, S. AND IMER, C.: "Contribution à l'étude des constituants du houblon (Partie I)" BULLETIN DE LA SOCIETE CHIMIQUE DE FRANCE, 1951, Seiten 634-637, XP002356398
- PARKHI, S.: "Synthesis of a completely reduced analogue of hexestrol, 1:2-diethyl-1-(4-methoxycyclohexyl)-2-(4-i soamyl-cyclohexyl)-ethane" JOURNAL INDIAN CHEMICAL SOCIETY, 1956, Seiten 313-317, XP008056361 in der Anmeldung erwähnt

## Beschreibung

Die vorliegende Erfindung betrifft primär die Verwendung von 4-Isoamylcyclohexanol [4-(3-Methylbutyl)-cyclohexan-1-ol], insbesondere von cis-4-Isoamylcyclohexanol, als Riech- oder Aromastoff, insbesondere als blumiger Riechstoff, welcher sich besonders durch Rosen- und Geranium-Noten auszeichnet. Die Erfindung betrifft daneben bestimmte Mischungen von cis- und trans-4-Isoamylcyclohexanol sowie ein Verfahren zur Herstellung dieser Mischungen. Ferner betrifft die vorliegende Erfindung entsprechende parfümierte Produkte.

In der Parfümindustrie besteht generell ein Bedarf an neuartigen und originellen Riechstoffen, da den Konsumenten laufend neue und moderne Düfte zur Verfügung gestellt werden sollen. Wegen der steigenden Nachfrage der Verbraucher nach neuen modernen Duftnoten, besteht in der Parfümindustrie ein ständiger Bedarf an blumigen Duftstoffen, mit denen sich in Parfüms neuartige Effekte erzielen und auf diese Art neue Modetrends kreieren lassen.

Für die Kreation neuartiger moderner Kompositionen besteht ständiger Bedarf an blumigen Riechstoffen mit besonderen geruchlichen Eigenschaften, die geeignet sind, als Grundlage für die Komposition von neuartigen modernen Parfüms zu dienen. Die gesuchten Riechstoffe sollen neben einem typischen blumigen Primärgeruch weitere Noten und Aspekte aufweisen, die ihnen geruchlichen Charakter, wie beispielweise Natürlichkeit und Strahlung, und Komplexität verleihen.

Die Suche nach geeigneten blumigen Riechstoffen, die zur vorliegenden Erfindung führte, wurde durch folgende Sachverhalte erschwert:
- Die Mechanismen der Geruchswahrnehmung sind nicht ausreichend bekannt.
- Die Zusammenhänge zwischen der speziellen Geruchswahrnehmung einerseits und der chemischen Struktur des zugehörigen Riechstoffs andererseits sind nicht hinreichend erforscht.
- Häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten blumigen Riechstoffen hängt deshalb stark von der Intuition des Suchenden ab.

Es war daher die Aufgabe der vorliegende Erfindung, blumige Riechstoffe mit neuen Geruchseigenschaften zu finden, mit welchen Riechstoffkompositionen besondere geruchliche Noten und Aspekte verliehen werden können.

Es wurde nun überraschenderweise gefunden, dass 4-Isoamylcyclohexanol geeignet ist, die gestellte Aufgabe zu lösen.

In S. Arctander, Perfume and Flavor Chemicals, Vol. I und II, Montclair, N. J., 1969, Selbstverlag werden diverse 4-Alkylcyclohexanole geruchlich beschrieben. 4-tert.-Amylcyclohexanol (Nummer 165) wird geruchlich als trocken, pinienartig-holzig mit guter Haftfestigkeit und beachtlicher Diffusivität charakterisiert. Unter der Nummer 433 wird der Geruch von p-tert.-Butylcyclohexanol als extrem trocken, holzig-camphrig, fast teerig mit ledrigen Aspekten beschrieben. 4-Ethylcyclohexanol (Nummer 1201) hingegen weist einen süßen, recht stechenden, gasigen Geruch auf, begleitet von blumigen Untertönen. Unter der Nummer 1531 wird p-Heptylcyclohexanol als camphrig-grüner, teils holziger, recht haftfester Riechstoff beschrieben. 4-Isopropylcyclohexanol (Nummer 2692) ist ein holzig-camphriger, pinienartiger Riechstoff mit süßen Untertönen und mäßiger Haftfestigkeit.

US 4,326,997 und US 4,400,545 betreffen diverse 4-Isoamylcyclohexanol-Derivate und deren Verwendung als Geruchs- oder Geschmackstoff, wobei die 2,6-Dimethyl-, 2,3,6-Trimethyl- und 2,3,5,6-Tetramethylderivate besonders hervorgehoben werden. Die geruchlichen Eigenschaften sämtlicher alicylcischer 4-Isoamylcyclohexanol-Derivate, inklusive aller Stereoisomeren und deren Mischungen (insbesondere Alkohole der Formel (IV) und (VI) sowie Ketone der Formeln (V), (IX) und (X)) werden dort pauschal als balsamisch, holzig, süß, wurzelig, muffig, erdig, ledrig, citrusartig und krautig beschrieben. Eine Isolierung und Charakterisierung der einzelnen cis- bzw. trans-Isomeren erfolgte nicht.

In EP 0 005 196 wird der Geruch von 4-Octylcyclohexanol als schwach holzig und leicht blumig beschrieben.

EP 0 053 979 betrifft Cyclopentanole und deren Derivate, insbesondere 3-Alkenylcyclopentan-1-ole und deren Ester. Als Riechstoffe mit rosigen Noten werden 3-(2-Butenyl-/Pentenyl-)cyclopentan-1-ole und insbesondere 1-Acetyloxy-3-(2-butenyl)cyclopentan beschrieben.

JP 02-131405 betrifft bestimmte Insektenrepellentien basierend auf 2,- 3, - und 4-Alkylcycloalkanolen und deren Estern. Es wird, unter Verweis auf die oben bereits genannten Stellen der Arctander Monographie, am Rande erwähnt, dass diese Insektenrepellentien für den Menschen unschädlich sind, da die eingesetzten Verbindungen als Riechstoffe geeignet sind. In der Anmeldung selbst werden keine Geruchsbeschreibungen angegeben. 4-Isoamylcyclohexanol wird nicht erwähnt.

WO 98/47842 betrifft unter anderem 3-Alkylcycloalkanole. Der Geruch des trans-3-Isoamylcyclohexan-1-ols wird als stark, fettig, Grapefruit, Rhabarber, Maiglöckchen, Rose und Citronellal charakterisiert, der des cis-3-Isoamylcyclohexan-1-ols hingegen als schwach, sehr fettig, blumig und nussig. Der Geruch einer Mischung aus cis- und trans-1-Methyl-3-(2-methylpropyl)cyclohexan-1-ol wird als stark blumig vom Maiglöckchen und Flieder Typ beschrieben, gepaart mit krautigen, pinienartigen Aspekten und Citrus Noten. Das reine trans-Isomere zeigt neben dem Maiglöckchenduft fruchtige und Rhabarber Noten, wohingegen das reine cis-Isomere daneben eher krautige und pinienartige Noten aufweist.

Die Synthese von 4-Isoamylcyclohexanol durch Hydrierung von p-Isoamylphenol unter Verwendung von Raney-Nickel ist in J. Indian. Chem. Soc. 1956, 33, 313-317 beschrieben. Eine geruchliche Beschreibung fehlt. Eigene Versuche haben ergeben, dass unter den angegebenen Versuchsbedingungen ein 4-Isoamylcyclohexanol mit einem lsomerenverhältnis cis- : trans-4-Isoamylcyclohexanol von etwa 33 : 67 erhalten wird. Es sei insoweit auch auf Beispiel 3.1 weiter unten verwiesen.

Es hat sich nun überraschenderweise gezeigt, dass sich 4-Isoamylcyclohexanol von in den oben genannten Dokumenten beschriebenen, strukturell verwandten Verbindungen geruchlich deutlich unterscheidet.

Die Geruchsbeschreibung der isolierten cis- und trans-Isomere von 4-Isoamylcyclohexanol und von Isomerenmischungen sind in Tabelle 1 dargestellt.

**Tabelle 1**

| Verbindung(en) | Geruchsbeschreibung |
|---|---|
| cis-4-Isoamylcyclohexanol | stark rosig mit leichter Geranium-Note, sehr natürlich , erinnert an Rosenöl, klar, transparent, ausstrahlender Geruch, sehr intensiv |
| trans-4-Isoamylcyclohexanol | rosig, Geranium, im Vergleich zum reinen cis-Isomeren schwächer mit weniger Ausstrahlung und Natürlichkeit |
| Mischung enthaltend 67,6 Gew.-% cis-4-Isoamylcyclohexanol und 31,8 Gew.-% trans-4-Isoamylcyclohexanol | rosig, animalisch, natürlich, indolig, Jasmin, geranienartig, klar, sehr intensiv |
| Mischung enthaltend 53,9 Gew.-% cis-4-Isoamylcyclohexanol und 44,9 Gew.-% trans-4-Isoamylcyclohexanol | animalisch, indolig, blumig, frisch rosig, geranienartig, sehr Intensiv |
| Mischung enthaltend 33,1 Gew.-% cis-4-Isoamylcyclohexanol und 66,3 Gew.-% trans-4-Isoamylcyclohexanol | rosig, citronellolartig, indolig, Flieder, Skatol, Jasmin, blumig, etwas Maiglöckchen, weniger intensiv |

Die sensorischen Untersuchungen zeigen somit, dass sich 4-Isoamylcyclohexanol signifikant von den in der Literatur angegebenen, teilweise sehr pauschalen Hinweisen zur sensorischen Bewertung strukturell verwandter Verbindungen unterscheidet.

Überraschenderweise unterscheidet sich das bisher als Riechstoff unbekannte 4-Isoamylcyclohexanol geruchlich deutlich von 3-Isoamylcyclohexanol insbesondere durch die parfümistisch begehrte intensive und wertvolle natürliche Rosen-und/oder Geranium-Note.

Bei dem erfindungsgemäß einzusetzenden 4-Isoamylcyclohexanol zeigt insbesondere das cis-Isomere einen besonders stark ausgeprägten Geruch nach Rosen mit Geranium-Note und einen sehr natürlichen, hellen und transparenten Geruch. Cis-4-Isoamylcyclohexanol erinnert an Rosenöl und verfügt über hohe Strahlung und Intensität. Ein Aspekt der vorliegenden Erfindung betrifft daher (aufgereinigtes) cis-4-Isoamylcyclohexanol.

Die Erfindung betrifft auch eine Mischung (a) umfassend oder bestehend aus cis- und trans-4-Isoamylcyclohexanol. Dabei ist das Gewichtsverhältnis von cis-4-Isoamylcyclohexanol zu trans-4-Isoamylcyclohexanol erfindungsgemäß größer als 2 : 3, bevorzugt größer oder gleich 1 : 1, besonders bevorzugt größer oder gleich 1,5 : 1 und und insbesondere bevorzugt größer oder gleich 2 : 1. Vorzugsweise ist das Gewichtsverhältnis von cis-4-Isoamylcyclohexanol zu trans-4-Isoamylcyclohexanol kleiner als 6 : 1, bevorzugt kleiner als 4 : 1.

Eine erfindungsgemäße Mischung aus cis- und trans-4-Isoamylcyclohexanol besitzt im Vergleich zu dem reinen cis- oder trans-4-Isoamylcyclohexanol eine wesentlich größere Intensität, Komplexität, Ausstrahlung, Natürlichkeit und Eleganz und ist daher besonders für die Verwendung in neuen und modernen Parfüm-Kompositionen geeignet.

Die Erfindung betrifft ferner (Riech- oder Aromastoff-)Mischungen umfassend oder bestehend aus (b) cis-4-Isoamylcyclohexanol und zumindest einem weiteren Riech- oder Aromastoff, wobei trans-4-Isoamylcyclohexanol nicht anwesend ist, oder (c) trans-4-Isoamylcyclohexanol und zumindest einem weiteren Riech-oder Aromastoff, wobei cis-4-Isoamylcyclohexanol nicht anwesend ist.

In Mischungen mit anderen Riechstoffen vermag der erfindungsgemäß einzusetzende 4-Isoamylcyclohexanol bereits in geringen Dosierungen die Intensität einer Riechstoffmischung zu verstärken und das Gesamtbild der Riechstoffmischung geruchlich abzurunden sowie der Mischung mehr Ausstrahlung sowie Natürlichkeit zu verleihen. In höheren Dosierungen kommt der kräftige rosenartige Geruch zum Tragen.

Die Erfindung betrifft zudem die Verwendung von 4-Isoamylcyclohexanol in Form (a) einer Mischung der cis/trans-Isomeren oder (b) des cis-4-Isoamylcyclohexanol oder (c) des trans-4-Isoamylcyclohexanol als Riech- oder Aromastoff, insbesondere die Verwendung einer cis/trans-Isomerenmischung in den oben angegebenen Gewichtsverhältnissen.

Ein entsprechendes Verfahren zum Vermitteln, Verstärken oder Modifizieren eines rosen- und/oder geraniumartigen Geruchs, umfasst die folgenden Schritte:
- Bereitstellen von 4-Isoamylcyclohexanol oder einer Mischung umfassend 4-Isoamylcyclohexanol, wobei das 4-Isoamylcyclohexanol jeweils vorliegt in Form (a) einer Mischung der cis/trans-Isomeren oder (b) des cis-4-Isoamylcyclohexanol oder (c) des trans-4-Isoamylcyclohexanol,
- Kontaktieren oder Vermischen einer sensorisch wirksamen Menge des 4-Isoamylcyclohexanol oder der Mischung umfassend 4-Isoamylcyclohexanol mit einem Erzeugnis.

Die Herstellung von 4-Isoamylcyclohexanol kann vorteilhaft aus γ,γ- Dimethyla I-lylphenylether durch Umlagerung und anschließende Hydrierung erfolgen.

γ,γ-Dimethylallylphenylether kann ausgehend von Phenol und Prenylchlorid in Anlehnung an die Vorschrift gemäß Helv. Chim. Acta 1968, 1603 in Gegenwart von Natriumhydroxid in N-Methylpyrrolidon bei 45°C hergestellt oder gemäß Synthesis, 1998, 256, in Gegenwart von Kaliumcarbonat in Aceton hergestellt werden.

Der so erhältliche γ,γ- Dimethylallylphenylether wird anschließend zu 4-(3-Methyl-but-2-enyl)-phenol umgelagert, wie beispielsweise wie in Helv. Chim. Acta 1968, 1603, oder in J. Org. Chem. 1976, 41, 3026, in Gegenwart von Diethylanilin oder Natriumacetat bei 200 bis 214°C, beschrieben.

Alternativ kann 4-(3-Methyl-but-2-enyl)-phenol auch durch Friedel-Crafts Alkylierung von Phenol und Dimethylvinynilcarbinol bzw. 3-Methyl-but-2-enol in Gegenwart von Säuren, wie z.B. von Phosphorsäure, synthetisiert werden. Eine derartige Herstellung wird in Zh. Org. Khim. (Engl. Trans) 1969, 1027 beschrieben.

Das 4-(3-Methyl-but-2-enyl)-phenol wird anschließend zu 4-Isoamylcyclohexanol hydriert.

Ein weiterer Gegenstand der Erfindung betrifft entsprechend ein Verfahren zur Herstellung von 4-Isoamylcyclohexanol in Form einer Mischung der cis/trans-Isomeren, mit folgendem Schritt:
- Hydrieren von 4-(3-Methyl-but-2-enyl)-phenol
   entweder (a) in Gegenwart von Rhodium oder Ruthenium, gegebenenfalls unter Zusatz einer Säure,
   oder (b) in Gegenwart eines Hydrierkatalysators der achten Nebengruppe und einer Säure.

Die erfindungsgemäßen Verfahren unterscheiden sich in der Wahl des Katalysators und/oder aufgrund der Anwesenheit von Säure von dem Herstellungsverfahren gemäß der oben angegebenen Veröffentlichung in J. Indian Chem. Soc.. Sie eignen sich zur Herstellung der erfindungsgemäßen Mischungen umfassend oder bestehend aus cis- und trans-4-Isoamylcyclohexanol, wobei das Gewichtsverhältnis von cis-4-Isoamylcyclohexanol zu trans-4-Isoamylcyclohexanol größer oder gleich 2 : 3, bevorzugt größer oder gleich 1 : 1, besonders bevorzugt größer oder gleich 1,5 : 1 und und insbesondere bevorzugt größer oder gleich 2 : 1 ist.

Die katalytische Hydrierung mit Wasserstoff erfolgt erfindungsgemäß im Regelfall in Gegenwart eines Hydrierkatalysators der achten Nebengruppe des Periodensystems, bevorzugte Hydrierkatalysatoren umfassen Palladium, Nickel, Platin, Rhodium oder Ruthenium, bevorzugt in elementarer Form. Die erfindungsgemäß eingesetzten Hydrierkatalysatoren können auf organische oder anorganische Trägermaterialien aufgebracht sein. Die Hydrierkatalysatoren können ein Trägermaterial oder Mischungen von Trägermaterialien enthalten. Als vorteilhafte Trägermaterialen seien Aktivkohle, Kohle, Aluminiumoxide, Metalloxide, Kieselgele, Zeolithe, Tone, Tongranulate oder amorphe Aluminiumsilicate genannt. Bevorzugte Katalysatoren sind Rhodium oder Ruthenium, bevorzugtes Trägermaterial ist Aktivkohle.

Die Hydrierung erfolgt gemäß der Alternative (b) des erfindungsgemäßen Verfahrens unter Zusatz von Säuren, wie beispielsweise Salzsäure, Salpetersäure, Schwefelsäure, Methansulfonsäure oder Phosphorsäure sowie Polymer geträgerte Säuren oder Harze, wie sie beispielsweise unter den Namen Amberlyst oder Lewatit kommerziell erhältlich sind. Gemäß der Alternative (a) des erfindungsgemäßen Verfahrens - bei Verwendung von Rhodium oder Ruthenium als Katalysator - kann auf die Anwesenheit von Säure verzichtet werden. Sofern die Hydrierung in Gegenwart einer Säure erfolgt, sind katalytische Mengen vorteilhaft, insbesondere 0,05 bis 20 Gew.-%, bevorzugt 1 bis 15 Gew.-%, Säure bezogen auf 4-(3-Methyl-but-2-enyl)-phenol. Vorteilhafte Säuren sind beispielsweise Salzsäure und Methansulfonsäure. Der Zusatz von Säure bei der Hydrierung bewirkt in den erfindungsgemäßen Verfahrensalternativen einen höheren Anteil des geruchlich wertvolleren cis-Isomeren.

Das Gewichtsverhältnis des eingesetzten Hydrierkatalysators (sofern zutreffend: inklusive Trägermaterial, ohne Wasseranteil) zu 4-(3-Methyl-but-2-enyl)-phenol liegt vorzugsweise im Bereich 1 : 10 000 bis 1 : 10, bevorzugt im Bereich 1 : 1 000 bis 1 : 10 und besonders bevorzugt im Bereich 1 : 100 bis 1 : 20.

Die Hydrierung kann in Verdünnungsmitteln wie z.B. Ethanol, Isopropanol, Essigester, Hexan, Cyclohexan, Pentan, Heptan oder auch verdünnungsmittelfrei, d.h. in Substanz, erfolgen. Besonders bevorzugt ist die verdünnungsmittelfreie Hydrierung sowie die Hydrierung in den Verdünnungsmitteln Hexan oder Cyclohexan.

Typischerweise erfolgt die Hydrierung bei einen Wasserstoffdruck im Bereich von 1 bis 200 bar.

Die Temperatur bei der Hydrierung liegt typischerweise im Bereich von 20 bis 200°C, bevorzugt im Bereich von 70 bis 180°C.

Die Reaktionszeit der Hydrierung beträgt in vielen Fällen vorteilhafterweise 1 bis 50 Stunden, bevorzugt 2 bis 15 Stunden.

Der GC-Gehalt (Gehalt im Gaschromatogramm) des gewünschten 4-Isoamylacyclohexanols im nach der Hydrierung vorliegenden Rohprodukt liegt bei Beachtung der vorstehend genannten Reaktionsparameter regelmäßig im Bereich von 90 bis 100 %.

Die Erfindung betrifft auch parfümierte Produkte umfassend einen festen oder halbfesten Träger und eine den festen bzw. halbfesten Träger kontaktierende sensorisch wirksame Menge einer erfindungsgemäßen Mischung, oder eine flüssige Phase und darin gelöst oder suspendiert eine sensorisch wirksame Menge einer erfindungsgemäßen Mischung.

Übliche sonstige Parfümbestandteile, mit denen 4-Isoamylcyclohexanol (in jeder der genannten Formen, also als cis-Isomer, trans-Isomer oder cis/trans-Isomerenmischung) vorteilhaft zu einer Riechstoffmischung (= Parfümöl-Komposition) kombiniert werden kann, finden sich z.B. in Steffen Arctander, Perfume and Flavor Chemicals, Eigenverlag, Montclair, N.J., 1969; K. Bauer, D. Garbe, H. Surburg, Common Fragrance and Flavor Materials, 4th Edition, Wiley-VCH, Weinheim 2001.

Im Einzelnen seien genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos - Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskatel-Ier-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-lonon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyl-dodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 7-Cyclohexadecen-1-on; 8-Cyclohexadecen-1-on; 6,7-Dihydro-1,1,2,3,3-pentamethyl-4(5H)-indanon; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexen-carbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Deca-hydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methyl-ethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.
4-Isoamylcyclohexanol (in einer der genannten Formen) enthaltende Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Für manche Anwendungen ist es vorteilhaft, 4-Isoamylcyclohexanol (in einer der genannten Formen) enthaltende Parfümöle (Riechstoffmischungen) an einem Trägerstoff adsorbiert einzusetzen, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer; Cellulose-basierende Stoffe, Zucker oder Kunststoffe wie PVC, Polyvinylacetate oder Polyurethane sein.

Für andere Anwendungen ist es vorteilhaft, 4-Isoamylcyclohexanol (in einer der genannten Formen) enthaltende Parfümöle mikroverkapselt, sprühgetrocknet, als Einschluß-Komplex oder als Extrusions-Produkt einzusetzen und in dieser Form dem zu parfümierenden (Vor-)Produkt hinzufügen.

Die Eigenschaften derart modifizierter Parfümöle werden in manchen Fällen durch sogenanntes "Coaten" mit geeigneten Materialien im Hinblick auf eine gezieltere Duftfreisetzung weiter optimiert, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

4-Isoamylcyclohexanol (in einer der genannten Formen) enthaltende Parfümöle können in konzentrierter Form, in Lösungen oder in sonstiger modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und -lotionen, Aftershave-Cremes und -lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder Produkten der dekorativen Kosmetik.

In Parfümöl-Kompositionen (= Riechstoffmischungen) liegt die eingesetzte Menge von 4-Isoamylcyclohexanol (in einer der genannten Formen) üblicherweise im Bereich von 0,001 bis 70 Gew.-%, vorzugsweise 0,05 bis 50 Gew.-% und besonders bevorzugt 0,5 bis 25 Gew.-%, bezogen auf die gesamte Parfümöl-Komposition.

Zutaten, mit denen 4-Isoamylcyclohexanol (in einer der genannten Formen) kombiniert werden können, sind beispielsweise:
Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Gesucht werden - insbesondere für die Parfümierung von tensidhaltigen Formulierungen, wie zum Beispiel für Shampoos, Waschmittel oder Weichspüler - häufig Riechstoffmischungen mit einer rosig-blumigen Kopfnote, wobei diese gleichzeitig ein ausgeprägtes Blooming (Geruch aus einer wässrigen Tensidlösung) aufweisen sollen. Eine weitere wichtige anwendungstechnische Anforderung an Riechstoffmischungen für tensidhaltige Produkte ist deren Substantivität gegenüber dem bzw. Retention am Substrat, insbesondere Haaren oder textilen Fasern.

4-Isoamylcyclohexanol zeichnet sich, insbesondere für einen blumigen Riechstoff, durch ein hohes Aufziehvermögen (Eigenhaftung auf einem Substrat) und eine hohe Substantivität (Fähigkeit aus einer, meist wässrigen, Phase heraus auf ein Substrat aufzuziehen bzw. auch nach einem Wasch- oder Spülvorgang auf einem Substrat zu verbleiben) aus. Dieser Effekt zeigt sich insbesondere auf Substraten wie Haut, Haar und textilen Fasern (z.B. Wolle, Baumwolle, Leinen, synthetische Fasern).

Im direkten Vergleich mit dem rosigen Riechstoff Citronellol (3,7-dimethyl-6-octan-1-ol) wurde beispielsweise eine deutlich höhere Eigenhaftung des 4-Isoamylcyclohexanols festgestellt. Die Riechstoffe wurden hierzu jeweils als 5%ige ethanolische Lösung auf einen Papier-Riechstreifen bzw. einen Baumwollstreifen aufgebracht und in festen Abständen abgerochen.

Überraschenderweise wird bei erfindungsgemäßer Verwendung des 4-Isoamylcyclohexanols (in einer der oben angegebenen Formen) in einer tensidhaltigen Formulierung eine (frisch) rosig-blumige (Kopf)Note, wobei auch interessante Jasmin- und Geranium-Nuancen sowie indolige und animalische Aspekte auftreten, nicht nur in Verbindung mit einer hohen Substantivität/Retention, sondern auch in Verbindung mit einem überraschenden Blooming (das ist der über einer tensidhaltigen wässrigen Lösung wahrgenommene Geruch) erreicht.

Durch Verwendung des 4-Isoamylcyclohexanols lässt sich in der Regel bereits in geringer Dosierung in einer resultierenden Parfümkomposition eine rosig-blumig Note mit einem ausgeprägten Blooming und einer erhöhten Substantivität für wässrige, tensidhaltige Anwendungen erzielen.

Besonders bevorzugte erfindungsgemäße parfümierte Produkte sind daher Waschmittel, Hygiene- oder Pflegeprodukte, insbesondere im Bereich der Körperpflege, der Kosmetik und des Haushalts.

Von großem Interesse für die parfümistische Komposition sind daneben Riechstoffe, die die Haftfestigkeit der Komposition verbessern (also als Fixateure wirken) oder die Stärke der geruchlichen Wahrnehmung erhöhen (also als Booster fungieren).

Neben Aufziehvermögen und Substantivität zeichnet sich 4-Isoamylcyclohexanol durch seine fixierenden Eigenschaften aus. Ein solcher Fixateur erhöht die Haftfestigkeit von anderen Riechstoffen, sei es durch deren Dampfdruckerniedrigung oder geruchlicher Verstärkung (z.B. Absenkung des Schwellenwertes). Die Erfindung betrifft daher auch die Verwendung von 4-Isoamylcyclohexanol (in einer der oben angegebenen Formen) (wie oben charakterisiert) als Fixateur.

Des Weiteren wirkt 4-Isoamylcyclohexanol (in einer der oben angebenen Formen) nicht nur als Fixateur, sondern auch als sogenannter Booster oder Enhancer, d.h. er bewirkt eine Verstärkung des Geruchs bzw. der geruchlichen Wahrnehmung von Riechstoffen, Riechstoffmischungen und Parfümkompositionen. Die Erfindung betrifft daher auch die Verwendung von 4-Isoamylcyclohexanol (in einer der oben angegebenen Formen) (wie oben charakterisiert) als Mittel zur Erhöhung der geruchlichen Wahrnehmung von Riechstoffen oder Riechstoffkompositionen.

Die folgenden Beispiele erläutern die Erfindung; sofern nicht anders angegeben beziehen sich Anteile und Prozente auf das Gewicht.

### Beispiele:

### Beispiel 1:

### Herstellung von Phenylprenylether [(3-Methyl-but-2-enyloxy)-benzol]

### Beispiel 1.1

Es werden 391 g (4,16 mol) Phenol, 532 g (4,16 mol) Kaliumcarbonat und 45 g Kaliumiodid in 1000 ml Aceton vorgelegt. Bei Raumtemperatur werden 478 g (4,5 mol) Prenylchlorid zugegeben. Anschließend wird das Reaktionsgemisch 4 bis 5 Stunden unter Rückfluss erhitzt. Nach beendeter Reaktion kühlt man ab und versetzt mit Wasser. Nach Phasentrennung wird die wässrige Phase dreimal mit Diethylether extrahiert. Die vereinigten organischen Phasen werden mit Kochsalzlösung gewaschen und über Na₂SO₄ getrocknet, filtriert und eingeengt. Das Rohprodukt wird destillativ gereinigt. Ausbeute an Phenylprenylether: 444 g; 66%d.Th.

¹H-NMR (400 MHz, CDCl₃): δ (ppm) = 1,74 (d, J = 0,9 Hz, 3H), 1,79 (q, J = 1,0Hz, 3H), 4,50 (dt, J = 6,82, 0,95 Hz, 2H), 5,50 (d sep., J = 6,82, 1,4 Hz, 1H), 6,84-7,04 (m, 3H), 7,22-7,34 (m, 2H).

¹³C-NMR (101 MHz, CDCl₃): δ (ppm) = 18.28, 25.94, 64.67, 114.66, 119.86, 120.61, 129.44, 137.99, 158.84.

### Beispiel 1. 2.

Zu einer gerührten Mischung aus 376,4 g (4,0 mol) Phenol und 172g (4,3 mol) Natriumhydroxid in 2400 g N-Methylpyrrolidon tropft man bei 45°C innerhalb von 30 min. 418 g (4,0 mol) Prenylchlorid zu. Anschließend wird das Reaktionsgemisch 1 Stunde bei dieser Temperatur nachgerührt. Der ausgefallene Festoff wird abfiltriert und mit t-Butylmethylether nachgewaschen. Das Rohprodukt wird destillativ gereinigt. Ausbeute an Phenylprenylether: 312 g; 83% d.Th.

### Beispiel 2:

### Umlagerung von Phenylprenylether zu 4-(3-Methyl-but-2-enyl)-phenol

### Beispiel 2.1.

In einem mit Kopfrührer versehenen Kolben werden 837 g (5,16 mol) Phenylprenylether und 459 g (5,16 mol) wasserfreies Natriumacetat in einer Schutzgasatmosphäre (Stickstoff) vorgelegt. Unter Rühren wird 6 bis 8 h auf 190-200°C erhitzt.

Nach beendeter Reaktion kühlt man ab und versetzt mit Wasser. Daraufhin werden die Phasen getrennt und die wässrige Phase dreimal mit 500 ml Diethylether extrahiert. Die vereinigten organischen Phasen werden über Na₂SO₄ getrocknet, filtriert und am Rotationsverdampfer eingeengt. Das Rohprodukt wird destillativ gereinigt. Ausbeute: 413 g ; 50% d.Th.

¹H-NMR (400 MHz, CDCl₃): δ (ppm) = 1.70 (dq, J = 1.0, 0.4 Hz, 3H), 1.73 (dq, J = 1.1, 0.4 Hz, 3H), 3.26 (d, J = 7,4 Hz, 2H), 5.29 (dsep., J = 7.4, 1.4 Hz, 1H), 4.70 (s, OH), 6.74-6.80 (m, 2H), 7.0-7.10 (m, 2H).

¹³C-NMR (101 MHz, CDCl₃): δ (ppm) = 17.72, 25.71, 33.38, 115.22 (2C), 123.46, 129.26 (2C), 132.10, 133.91, 153.12

### Beispiel 2.2.

In einem mit Kopfrührer versehenen Kolben werden 245,8 (1,5 mol) (3-Methylbut-2-enyloxy)benzol und 1220 ml n-Diethylanilin vorgelegt. Unter Rühren wird 6h auf 214°C erhitzt.

Die so erhaltene Mischung wird ohne Aufarbeitung destillativ gereinigt. Ausbeute: 171 g ; 70% d.Th.

### Beispiel 3:

### Hydrierung von 4-(3-Methyl-but-2-enyl)-phenol zu 4-Isoamylcyclohexanol

### Beispiel 3.1

### (nicht erfindungsgemäßes Herstellverfahren, J. Indian. Chem. Soc. 1956, 33, 313-317)

In einen Rührautoklaven mit Begasungsrührer werden 165 g 4-(3-Methyl-but-2-enyl)-phenol, 2 Gew.-% Raney-Nickel-Eisen (Zusammensetzung des Katalysators: 45 % Nickel, 3 % Aluminium, 8 % Eisen; Wassergehalt 44 %) vorgelegt. Es wird 6 Stunden bei 170°C und einem Wasserstoffdruck von 20 bar hydriert. Nach Filtration erhält man 165 g Rohprodukt. Durch Destillation des Rohprodukts werden 158 g 4-Isoamylcyclohexanol (Sdp.: 65°C, 0,6 mbar) erhalten, welches folgende Zusammensetzung aufweist 33,1% cis-4-Isoamylcyclohexanol und 66,3% trans-4-Isoamylcyclohexanol.

500 mg (GC-Reinheit > 98%) des 4-Isoamylcyclohexanols wurden präparativ mittels HPLC aufgetrennt. Man erhält 160 mg cis-4-Isoamylcyclohexanol mit einer GC-Reinheit > 98% und 180 mg trans-4-Isoamylcyclohexanol mit einer GC-Reinheit > 98%. HPLC-Bedingungen: Säule Grom Saphir 110 Si, 5µm, 125x20 mm, Eluent: Heptan/t-Butylmethylether (v/v) 60:40, Fluss: 16 ml/min, Druck: 14 bar, Temperatur: RT, Detektion: RI zusätzliche Detektion: UV bei 210 nm.

Cis-4-Isoamvlcyclohexanol: Unter der Annahme, dass der sperrige Alkylrest eine equatoriale Lage einnimmt und die OH-Gruppe sich in axialer Lage befindet.

¹H-NMR (400 MHz, CDCl₃): δ (ppm) = 0.87 (d, J = 6,6 Hz, 6H), 1.15-1.40 (m, 1.66-1.74 , 7H), 3.95 (tt, J_{eq,ax} = 4,7Hz, J = 3,95 Hz, 1 H).

¹³C-NMR (101 MHz, CDCl₃): δ (ppm) = 22.68 (2C), 27.10, 28.23, 32.30, 33.70 (2C), 36.37, 36.46, 67.34.

MS m/z (%): 169 (1[M-H]⁺), 152 (8 [M-H₂O]), 137 (4), 124 (4), 109 (14), 96 (83), 81 (100), 71 (25), 67 (23), 57 (52), 43 (30).

Trans-4-Isoamylcylohexanol: Unter der Annahme, dass der sperrige Alkylrest eine equatoriale Lage einnimmt und die OH-Gruppe sich in equatorialer Lage befindet.

¹H-NMR (400 MHz, CDCl₃): δ (ppm) = 0.86 (d, J = 6,6 Hz, 6H), 0.90-1.10 (m, 2H), 1.10-1.30 (m, 7H), 1.42-1.52 (m, 2H), 1.721.80 (m, 2H), 1.92-2.0 (m, 2H), 3.54 (tt, J_{ax,ax} = 10,9Hz, J = 4.3 Hz, 1 H).

¹³C-NMR (101 MHz, CDCl₃): δ (ppm) = 22.65 (2C), 28.23, 31.34(2C), 34.40, 35.65 (2C), 36.52, 36.70, 71.25.

MS m/z (%): 169 (1 [M-H]⁺), 152 (11 [M-H₂O]), 137 (5), 124 (4), 109 (16), 96 (99), 81 (100), 71 (46), 67 (32), 57 (76), 43 (44).

### Beispiel 3.2.

In einen Rührautoklaven mit Begasungsrührer werden 330 g 4-(3-Methyl-but-2-enyl)-phenol, 3 Gew.-% Ruthenium auf Aktivkohle (Ru-Gehalt bezogen auf das Gewicht des trockenen Katalysators: 5 %; Wassergehalt etwa 50 %) in 570 ml Isopropanol vorgelegt. Es wird 5 Stunden bei 100°C und einem Wasserstoffdruck von 130 bar hydriert. Nach Filtration erhält man 329 g 4-Isoamylcyclohexanol als Rohprodukt. Nach Destillation erhält man 315 g 4-Isoamylcyclohexanol, welches folgende Zusammensetzung aufweist: 53,8% cis-4-Isoamylcyclohexanol und 44,9% trans-4-Isoamylcyclohexanol.

### Beispiel 3.3.

In einen Rührautoklaven mit Begasungsrührer werden 85 g 4-(3-Methyl-but-2-enyl)-phenol, 3 Gew.-% Rhodium auf Aktivkohle (Rh-Gehalt bezogen auf das Gewicht des trockenen Katalysators: 5 %; Wassergehalt etwa 50 %) in 300 ml Isopropanol und 9,6 g Methansulfonsäure vorgelegt. Es wird 5 Stunden bei 80°C und einem Wasserstoffdruck von 10 bar hydriert. Nach Filtration erhält man 85 g 4-Isoamylcyclohexanol als Rohprodukt. Nach Destillation erhält man 80 g 4-Isoamylcyclohexanol, welches folgende Zusammensetzung aufweist: 67,6% cis-4-Isoamylcyclohexanol und 31,8% trans-4-Isoamylcyclohexanol.

### Beispiel 3.4.

In einen Rührautoklaven mit Begasungsrührer werden 85 g 4-(3-methyl-but-2-enyl)-phenol, 3 Gew.-% Rhodium auf Aktivkohle (Rh-Gehalt bezogen auf das Gewicht des trockenen Katalysators: 5 %; Wassergehalt etwa 50 %) in 300 ml Cyclohexan vorgelegt. Es wird 5 Stunden bei 100°C und einem Wasserstoffdruck von 20 bar hydriert. Nach Filtration erhält man 85 g 4-Isoamylcyclohexanol als Rohprodukt. Nach Destillation erhält man 80 g 4-Isoamylcyclohexanol, welches folgende Zusammensetzung aufweist: 50,4% cis-4-Isoamylcyclohexanol und 49,1% trans-4-Isoamylcyclohexanol.

Geruch: Frisch rosig, intensiv, blumig, geranienartig, indolig, animalisch.

### Beispiel 4:

Herstellung eines Parfümöles mit einer modernen, betont blumig-rosigen Note.

Es werden die folgenden Riechstoffe in den angegebenen Mengen (Gewichtsteile) vermischt:

| | |
|---|---|
| Acetessigsäureethylester | 13 |
| Hexenylacetat, cis-3- | 1,5 |
| Vertocitral, Symrise | 1 |
| Hexenol, cis-3- | 0,5 |
| Isoananat, Symrise | 5 |
| Styrolylacetat | 8 |
| Leguminal, Symrise | 0,2 |
| Dihydromyrcenol | 11 |
| Mandarinenöl | 5 |
| Menthol I-, Symrise | 3 |
| Aldehyd C14 sogen. | 1 |
| Lilial, Givaudan | 65 |
| Bourgeonal, Quest | 3 |
| Helional, IFF | 3 |
| Florhydral, Givaudan | 10 |
| Mugetanol, Symrise | 50 |
| Lyral, IFF | 57 |
| Ethyllinalool, Givaudan | 102 |
| Dimethylbenzylcarbinylacetat | 3 |
| Phenylethylalkohol | 20 |
| Geraniol | 10 |
| Cirtronellol | 18 |
| Citronellylacetat | 7 |
| Geranylacetat | 4 |
| Damascon alpha, Firmenich | 0,5 |
| Benzylacetat | 14 |
| Methyldihydrojasmonat | 158 |
| Hexenylsalicylat, cis-3- | 4 |
| Iraldein gamma, Symrise | 7 |
| Cumarin | 0,3 |
| Iso E Super, IFF | 18 |
| Patchoulyöl | 4 |
| Sandolen, Symrise | 3 |
| Ambroxid krist., Symrise | 1 |
| Globalid, Symrise | 18 |
| Ethylenbrassylat | 21 |
| Summe | 650 |

Diese Ausgangskomposition ist ein Parfümöl mit einem grünen, blumigen Akkord mit betonter Maiglöckchen- und Rosennote.

Durch Zugabe von 100 Gewichtsteilen 4-Isoamylcyclohexanol (aus Beispiel 3.4) auf 650 Gewichtsteile der Ausgangskomposition wird ein modernes Parfüm mit einer ausgeprägten Fantasie-Rosennote erhalten. Im Vergleich zur Ausgangskomposition riecht die 4-Isoamylcyclohexanol enthaltende erfindungsgemäße Riechstoffkomposition feiner, blumig-rosiger, harmonischer und abgerundeter. Durch die Zugabe von 4-Isoamylcyclohexanol werden die Ausstrahlung und räumliche Wirkung der Ausgangskomposition wesentlich erhöht.

### Beispiele 5 bis 7: Vergleichsbeispiele

Bei den im Folgenden gezeigten Vergleichsexperimenten wurde eine erfindungsgemäße cis/trans-4-Isoamylalkohol-Isomerenmischung (Zusammensetzung gemäß Beispiel 3.2) im Vergleich zu nicht erfindungsgemäßen Citronellol in verschiedenen Hygiene- und Waschprodukten sowohl durch mehrere Experten als auch durch eine Gruppe von 16 Laienprüfern geprüft und hierbei wesentliche geruchliche Unterschiede festgestellt.

### Beispiel 5: Waschpulver (Powder detergent)

Jeweils 80 g Waschpulver (Zusammensetzung: 9% C₁₂-C₁₃ lineare Na-Alkylbenzolsulfonate, 1,6% C₁₄-C₁₅ Na -Alkylethoxysulfat (EO=0.6), 5,7% C₁₂-C₁₈ Alkylsulfate, 3,3% Polyacrylat (MW = 4.500, 27% Aluminosilicat, 0,6% Natriumsilicat, 28% Natriumcarbonat, 9% Natriumsulfat, 0,2% optischer Aufheller, 1,8% Polyethylenglykol (MW = 4.000), 1% Perborat, 1,1% Enzyme (Lipase, Protease, Cellulase), Wasser q.s.) wurden mit 1 g einer 50%igen Lösung in Isopropylmyristat (a) einer erfindungsgemäßen 4-Isoamylcyclohexanol-Mischung bzw. (b) Citronellol vermischt und die Waschpulver 24 Stunden bei Raumtemperatur gelagert. Anschließend wurden jeweils zwei Handtücher aus Baumwolle und zwei Handtücher aus Mischgewebe separat bei 40°C in Waschmaschinen (Hersteller: Miele) mit den jeweils 80 g Waschpulver gewaschen.

Die so erhaltene feuchte Wäsche wurde geruchlich untersucht und gefunden, dass die erfindungsgemäße 4-Isoamylcyclohexanol-Mischung eine deutlich höhere Duftintensität aufwies.

Abschließend wurden die einzelnen Handtücher für 24 Stunden auf einer Leine getrocknet. Die parfümistische Bewertung der beiden Handtücher durch eine Expertengruppe ergab eine deutliche höhere Duftintensität für die erfindungsgemäße 4-Isoamylcyclohexanol-Mischung. Eine Prüfergruppe mit 16 Laienprüfern bevorzugte in einer verdeckten Evaluierung die Handtücher mit der erfindungsgemäßen 4-Isoamylcyclohexanol-Mischung eindeutig mit signifikantem Unterschied (p < 0.05).

### Beispiel 6: Weichspüler (Fabric softener)

Jeweils 40 g eines Weichspülers (3-fach Konzentrat, 94% Trinkwasser, 5,5% Quartäre Ethanolaminester (Esterquats; Quarternary ammonium methosulfate), 0,2% Alkyldimethylbenzylammoniumchlorid (Preventol^{®} R50, Bayer AG) und 0,3% einer blauen Farbstoff-Lösung) wurden mit 0,5 g einer 50%igen Lösung in Isopropylmyristat (a) einer erfindungsgemäßen 4-Isoamylcyclohexanol-Mischung bzw. (b) Citronellol gut vermischt und die Weichspüler 24 Stunden bei Raumtemperatur gelagert. Der pH-Wert des Weichspülerkonzentrates liegt typischerweise im Bereich 2 - 3. Anschließend wurden jeweils drei Handtücher aus Baumwolle und drei Handtücher aus Mischgewebe bei 40°C in Waschmaschinen (Hersteller: Miele) zuerst mit 80 g eines unparfümierten Standardwaschpulvers und nachfolgend separat mit den zu untersuchenden Weichspülern gewaschen.

Die so erhaltene feuchte Wäsche wurde für 24 Stunden auf der Leine getrocknet. Die geruchliche Evaluierung der trockenen Handtücher ergab, dass die erfindungsgemäße 4-Isoamylcyclohexanol-Mischung eine deutlich höhere Duftintensität aufwies.

Die folgende Tabelle zeigt die Ergebnisse für die Intensität und die Präferenz nach der jeweiligen Bewertung der trockenen Handtücher nach der Behandlung mit den jeweiligen Weichspülern, jeweils auf einer Skala von 1 (= sehr schwach bzw. nicht gefällig) bis 6 (= sehr stark bzw. sehr gefällig). In beiden Fällen war die statistische Bewertung signifikant (p < 0.05).

| Mischung | Intensität | Präferenz |
|---|---|---|
| 4-Isoamylcyclohexanol | 3,0 | 3,7 |
| Citronellol | 2,6 | 3,0 |

### Beispiel 7: Shampoo

Jeweils 30 g eines Shampoos (20% Plantacare^{®} PS 10 (Cognis GmbH, Na-Laureth sulfat und Laurylglycosid), 2% Natriumchlorid, 1,3% Citronensäure, 0,5% Dragocid^{®} Liquid (Symrise GmbH & Co. KG, Mischung von Phenoxyethanol, Methyl-, Ethyl- Propyl- und Butylparaben), 76,2% Wasser) wurden mit 0,3 g einer 50%igen Lösung in Isopropylmyristat (a) einer erfindungsgemäßen 4-Isoamylcyclohexanol-Mischung bzw. (b) Citronellol gut vermischt und die Shampoos 24 Stunden bei Raumtemperatur gelagert. Der pH-Wert des Shampoos lag bei etwa 6. Anschließend wurde jeweils eine 20 g - Haarsträhne (Echthaar) mit 1 g des jeweiligen Shampoos, welches in 2 g Wasser aufgeschäumt wurde, für 1 Minute separat von Hand gewaschen. Zum Schluss wurden beide Haarsträhnen separat 30 Sekunden unter 30°C warmem Wasser ausgespült.

Die so erhaltenen feuchten Haarsträhnen wurde für 1 Minute mit einem elektrischen Haartrockner auf mittlerer Stufe getrocknet. Die geruchliche Evaluierung der Haarsträhnen ergab, dass die erfindungsgemäße 4-Isoamylcyclohexanol-Mischung sowohl auf feuchtem und trockenem Haar intensiver wahrgenommen als auch geruchlich bevorzugt wurde.

Die folgende Tabelle zeigt die Ergebnisse für die Intensität und die Präferenz nach der jeweiligen Bewertung der trockenen bzw. feuchten Haarsträhnen nach der Behandlung mit den Weichspülern, jeweils auf einer Skala von 1 (= sehr schwach bzw. nicht gefällig) bis 6 (= sehr stark bzw. sehr gefällig). In allen Fällen war die statistische Bewertung hochsignifikant (p < 0.01).

| Mischung | Feuchte Haarsträhne | | Trockene Haarsträhne | |
|---|---|---|---|---|
| | Intensität | Präferenz | Intensität | Präferenz |
| 4-Isoamylcyclohexanol | 3,0 | 3,6 | 2,3 | 3,8 |
| Citronellol | 2,8 | 3,0 | 2,0 | 3,1 |

## Patentansprüche

1. cis-4-Isoamylcyclohexanol.

2. Mischung umfassend oder bestehend aus,
(a) cis-4-Isoamylcyclohexanol und trans-4-Isoamylcyclohexanol, wobei das Gewichtsverhältnis von cis-4-Isoamylcyclohexanol zu trans-4-Isoamylcyclohexanol größer ist als 2 : 3, oder
(b) cis-4-Isoamylcyclohexanol und zumindest einem weiteren Riech- oder Aromastoff, wobei trans-4-Isoamylcyclohexanol nicht anwesend ist, oder
(c) trans-4-Isoamylcyclohexanol und zumindest einem weiteren Riech- oder Aromastoff, wobei cis-4-Isoamylcyclohexanol nicht anwesend ist.

3. Verwendung von 4-Isoamylcyclohexanol in Form
(a) einer Mischung der cis/trans-Isomeren oder
(b) des cis-4-Isoamylcyclohexanol oder
(c) des trans-4-Isoamylcyclohexanol
als Riech- oder Aromastoff.

4. Verfahren zum Vermitteln, Verstärken oder Modifizieren eines rosen-und/oder geraniumartigen Geruchs, mit folgenden Schritten:
- Bereitstellen von 4-Isoamylcyclohexanol oder einer Mischung umfassend 4-Isoamylcyclohexanol, wobei das 4-Isoamylcyclohexanol jeweils vorliegt in Form
(a) einer Mischung der cis/trans-Isomeren oder
(b) des cis-4-Isoamylcyclohexanol oder
(c) des trans-4-Isoamylcyclohexanol,
- Kontaktieren oder Vermischen einer sensorisch wirksamen Menge des 4-Isoamylcyclohexanol oder der Mischung umfassend 4-Isoamylcyclohexanol mit einem Erzeugnis.

5. Parfümiertes Produkt umfassend
(a) einen festen oder halbfesten Träger und eine den festen bzw. halbfesten Träger kontaktierende sensorisch wirksame Menge einer Mischung nach Anspruch 2,
oder
(b) eine flüssige Phase und darin gelöst oder suspendiert eine sensorisch wirksame Menge einer Mischung nach Anspruch 2.

6. Parfümiertes Produkt nach Anspruch 5, wobei das Produkt ein Waschmittel, Hygiene- oder Pflegeprodukt ist.

7. Verwendung von 4-Isoamylcyclohexanol in Form
(a) einer Mischung der cis/trans-Isomeren oder
(b) des cis-4-Isoamylcyclohexanol oder
(c) des trans-4-Isoamylcyclohexanol
als Fixateur oder als Mittel zur Erhöhung der geruchlichen Wahrnehmung von Riechstoffen oder Riechstoffkompositionen.

8. Verfahren zur Herstellung von 4-Isoamylcyclohexanol in Form einer Mischung der cis/trans-Isomeren, mit folgendem Schritt:
- Hydrieren von 4-(3-Methyl-but-2-enyl)-phenol
entweder (a) in Gegenwart von Rhodium oder Ruthenium, gegebenenfalls unter Zusatz einer Säure,
oder (b) in Gegenwart eines Hydrierkatalysators der achten Nebengruppe und einer Säure.

## Claims

1. Cis-4-isoamylcyclohexanol.

2. Mixture comprising or consisting of,
(a) cis-4-isoamylcyclohexanol and trans 4-isoamylcyclohexanol, the weight ratio of cis-4-isoamylcyclohexanol to trans-4-isoamylcyclohexanol being greater than 2 : 3, or
(b) cis-4-isoamylcyclohexanol and at least one further odiferous or aroma substance, trans-4-isoamylcyclohexanol not being present, or
(c) trans-4-isoamylcyclohexanol and at least one further odiferous or aroma substance, cis-4-isoamylcyclohexanol not being present.

3. Use of 4-isoamylcyclohexanol in the form of
(a) a mixture of the cis/trans isomers or
(b) cis-4-isoamylcyclohexanol or
(c) trans-4-isoamylcyclohexanol
as an odiferous or aroma substance.

4. Method for imparting, reinforcing or modifying a rose-like and/or geranium-like smell, having the following steps:
- provision of 4-isoamylcyclohexanol or a mixture comprising 4-isoamylcyclohexanol, the 4-isoamylcyclohexanol in each case being present in the form of
(a) a mixture of the cis/trans isomers or
(b) cis-4-isoamylcyclohexanol or
(c) trans-4-isoamylcyclohexanol,
- bringing into contact or mixing of a sensorially active amount of 4-isoamylcyclohexanol or the mixture comprising 4-isoamylcyclohexanol with a product.

5. Perfumed product, comprising
(a) a solid or semi-solid carrier and a sensorially active amount of a mixture according to Claim 2, in contact with the solid or semi-solid carrier,
or
(b) a liquid phase and a sensorially active amount of a mixture according to Claim 2 dissolved or suspended therein,

6. Perfumed product according to Claim 5, wherein the product is a detergent or a hygiene or care product.

7. Use of 4-isoamylcyclohexanol in the form of
(a) a mixture of the cis/trans isomers or
(b) cis-4-isoamylcyclohexanol or
(c) trans-4-isoamylcyclohexanol
as a fixative or as an agent for increasing the olfactory perception of odiferous substances or odiferous substance compositions.

8. Method for the preparation of 4-isoamylcyclohexanol in the form of a mixture of the cis/trans isomers, having the following step:
- Hydrogenation of 4-(3-methyl-but-2-enyl)-phenol
either (a) in the presence of rhodium or ruthenium, optionally with the addition of an acid,
or (b) in the presence of a hydrogenation catalyst of sub-group eight and an acid.

## Revendications

1. Cis-4-isoamylcyclohexanol.

2. Mélange comprenant ou constitué,
(a) du cis-4-isoamylcyclohexanol et du trans-4-isoamylcyclohexanol, le rapport massique du cis-4-isoamylcyclohexanol au trans-4-isoamylcyclohexanol étant supérieur à 2:3, ou
(b) du cis-4-isoamylcyclohexanol et au moins une autre matière odorante ou d'arôme, le trans-4-isoamylcyclohexanol n'étant pas présent, ou
(c) du trans-4-isoamylcyclohexanol et au moins une autre matière odorante ou d'arôme, le cis-4-isoamylcyclohexanol n'étant pas présent.

3. Utilisation de 4-isoamylcyclohexanol dans la forme
(a) d'un mélange des isomères cis/trans ou
(b) du cis-4-isoamylcyclohexanol ou
(c) du trans-4-isoamylcyclohexanol
comme matière odorante ou d'arôme.

4. Procédé pour obtenir, renforcer ou modifier une odeur de type rose et/ou géranium avec les étapes suivantes :
- de préparation de 4-isoamylcyclohexanol ou d'un mélange comprenant du 4-isoamylcyclohexanol, le 4-isoamylcyclohexanol étant à chaque fois présent dans la forme
(a) d'un mélange des isomères cis/trans ou
(b) du cis-4-isoamylcyclohexanol ou
(c) du trans-4-isoamylcyclohexanol,
- de mise en contact ou de mélange d'une quantité sensoriellement active du 4-isoamylcyclohexanol ou du mélange comprenant du 4-isoamylcyclohexanol avec un produit.

5. Produit parfumé comprenant
(a) un support solide ou semi-solide et une quantité sensoriellement active en contact avec le support solide respectivement semi-solide d'un mélange selon la revendication 2,
ou
(b) une phase liquide et dissoute ou mise en suspension dans celle-ci une quantité sensoriellement active d'un mélange selon la revendication 2.

6. Produit parfumé selon la revendication 5, le produit étant un détergent, un produit d'hygiène ou de soin.

7. Utilisation de 4-isoamylcylohexanol dans la forme
(a) d'un mélange des isomères cis/trans ou
(b) du cis-4-isoamylcyclohexanol ou
(c) du trans-4-isoamylcyclohexanol
comme fixateur ou comme agent pour augmenter la perception odorante de matières odorantes ou de compositions de matières odorantes.

8. Procédé pour la préparation de 4-isoamylcyclohexanol dans la forme d'un mélange des isomères cis/trans avec l'étape suivante :
- d'hydrogénation de 4-(3-méthyl-but-2-ényl)phénol
soit (a) en présence de rhodium ou de ruthénium, éventuellement en ajoutant un acide,
ou (b) en présence d'un catalyseur d'hydrogénation du huitième groupe secondaire et d'un acide.
